(19)

**Europäisches Patentamt**
**European Patent Office**
**Office européen des brevets**

(11) **EP 3 624 594 B1**

(12) **EUROPEAN PATENT SPECIFICATION**

(45) Date of publication and mention
of the grant of the patent:
**25.03.2026 Bulletin 2026/13**

(21) Application number: **18725466.9**

(22) Date of filing: **15.05.2018**

(51) International Patent Classification (IPC):
*A23C 9/12* (2006.01)   *A23C 9/13* (2006.01)
*C12N 9/38* (2006.01)   *C12N 9/26* (2006.01)
*A61K 31/702* (2006.01)   *C07H 3/06* (2006.01)
*C12P 19/14* (2006.01)   *C12P 19/04* (2006.01)
*C12P 19/18* (2006.01)

(52) Cooperative Patent Classification (CPC):
(C-Sets available)
**C12N 9/2471; A23C 9/1206; A23C 9/1307;
A61K 31/702; C12P 19/04; C12P 19/18;
C12Y 302/01023; C12Y 302/01108**   (Cont.)

(86) International application number:
**PCT/EP2018/062542**

(87) International publication number:
**WO 2018/210821 (22.11.2018 Gazette 2018/47)**

(54) **MILK PRODUCTS COMPRISING HIGH AMOUNTS OF IN SITU PRODUCED GALACTOOLIGOSACCHARIDES (GOS)**

MILCHPRODUKTE ENTHALTEND EINE HOHE KONZENTRATION VON IN SITU PRODUZIERTEN
GALAKTOOLIGOSACCHARIDEN (GOS)

PRODUITS LAITIERS COMPRENANT DES QUANTITÉS ÉLEVÉES DE
GALACTO-OLIGOSACCHARIDES (GOS) PRODUITS IN SITU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.05.2017 EP 17171174
16.03.2018 EP 18162193**

(43) Date of publication of application:
**25.03.2020 Bulletin 2020/13**

(73) Proprietor: **Kerry Group Services International
Ltd
Tralee, Co. Kerry, V92 EH11 (IE)**

(72) Inventor: **TAMS, Jeppe Wegener
2880 Bagsvaerd (DK)**

(74) Representative: **Bird & Bird Società tra Avvocati
S.r.l.
Via Porlezza, 12
20123 Milano (IT)**

(56) References cited:
**EP-A1- 0 458 358       EP-A1- 2 982 760
EP-A2- 1 283 876       WO-A1-2008/037839
WO-A1-2009/071539       WO-A1-2011/093907
WO-A1-2012/160080       WO-A1-2013/182686
WO-A2-2010/098561       CN-A- 101 103 743
CN-A- 101 831 389       JP-A- H1 118 763
KR-A- 20150 116 114       KR-B1- 101 121 161
US-A1- 2012 040 051**

• **JINGYUAN SONG ET AL: "The Discoidin Domain
of Bacillus circulans &bgr;-Galactosidase Plays
an Essential Role in Repressing
Galactooligosaccharide Production",
BIOSCIENCE BIOTECHNOLOGY
BIOCHEMISTRY, vol. 77, no. 1, 23 January 2013
(2013-01-23), JP, pages 73 - 79, XP055331755,
ISSN: 0916-8451, DOI: 10.1271/bbb.120583**

(Cont. next page)

- **Z. MOZAFFAR ET AL: "Formation of Oligosaccharides During Hydrolysis of Lactose in Milk Using beta-Galactosidase from Bacillus circulans", JOURNAL OF FOOD SCIENCE, vol. 50, 1 January 1985 (1985-01-01), pages 1602 - 1606, XP055819427**
- **DANIEL OBED OTIENO: "Synthesis of beta-Galactooligosaccharides from Lactose Using Microbial beta-Galactosidases", COMPREHENSIVE REVIEWS IN FOOD SCIENCE AND FOOD SAFETY, vol. 9, no. 5, 26 August 2010 (2010-08-26), pages 471 - 482, XP055819433**
- **CARLOS VERA ET AL: "Determination of the transgalactosylation activity of Aspergillus oryzae [beta]-galactosidase: effect of pH, temperature, and galactose and glucose concentrations", CARBOHYDRATE RESEARCH, vol. 346, no. 6, 4 February 2011 (2011-02-04), GB, pages 745 - 752, XP055242416, ISSN: 0008-6215, DOI: 10.1016/j.carres.2011.01.030**

Remarks:
The complete document including Reference Table(s) and the Sequence Listing(s) can be downloaded from the EPO website

(52) Cooperative Patent Classification (CPC): (Cont.)

C-Sets
**C12Y 302/01023, C12Y 302/01108**

## Description

### SEQUENCE LISTING

The present invention comprises a sequence listing.

### TECHNICAL FIELD

The present invention relates to milk products comprising a high amount of *in situ* produced galactooligosaccharides (GOS).

### BACKGROUND OF THE INVENTION

Galactooligosaccharides (GOS) can be produced from lactose through the enzymatic transgalactosylation reaction of beta-galactosidase. It has been shown that GOS can promote the growth of bifidobacteria, healthy microbes, in the large intestine of humans. Therefore, it is beneficial and desirable to produce milk products comprising high amounts of GOS.

It has been reported that the production of GOS from lactose increases with the concentration of lactose.

GOS can be produced *ex-situ* from solutions of lactose and added to dairy products as an ingredient. This will add calories to the dairy product, and further the addition of GOS is to be labelled on the final product. This goes against the general "clean label" trend, *i.e.* the preference of many consumers of "natural" products comprising only ingredients which are perceived as natural with no or few additives.

It is therefore preferred to produce the GOS *in-situ, i.e.* to make the GOS in the milk product from the lactose already present in the milk. Producing GOS *in situ* at high levels has been difficult though.

In EP 0458358 A1, skim milk is concentrated to a total solid content of 20-50% by weight. By incubating the concentrated milk with beta-galactosidase at a temperature of 20-50°C followed by spray-drying, it is speculated that skim milk powder containing a total amount of about 5-15% by weight of GOS (presumably DP2+) can be obtained. Assuming a total content of free carbohydrates in the concentrated skim milk (lactose and derivatives) of about 50% of the dry matter, this corresponds to a relative amount of 10-30% DP2+ GOS w/w of the total free carbohydrates. Production of skim milk powder comprising a total amount of 10.5% DP2+ GOS w/w is exemplified.

Chen et al. (2002), "Optimization of the enzymic process for manufacturing low-lactose milk containing oligosaccharides", Process Biochemistry 38, 801-808, have compared use of beta-galactosidase to transform the lactose in milk into GOS directly with the application of ultrafiltration techniques to separate lactose from milk proteins and then transform the lactose in the permeate into GOS. When using concentrated milk containing 16.7% lactose, a milk product could be obtained by incubating at 47°C for 1.4 h wherein 22.8% w/w of the lactose was converted to DP3+ GOS. Whereas by removing milk proteins by ultrafiltration and thereby increasing the lactose concentration in the permeate to 25.3% by evaporation, a permeate could be obtained by incubating at 50°C for 3.5 h wherein as much as 31.1% w/w of the lactose was converted to DP3+ GOS.

WO 2015/086746 discloses efficient conversion of low-concentration lactose (at most 15% w/v) to GOS by treatment of milk-based media comprising, e.g., skim milk, cream and lactose with a truncated beta-galactosidase from *Bifidobacterium bifidum* consisting of 887 amino acids. Disclosed is also a milk-based substrate which comprises GOS after the enzymatic treatment in a total amount of 0.1 to 10% (w/v). The examples show production of GOS from milk-based media to which lactose has been added. Incubation was at 45°C or lower and a total amount of up to 3.7% (w/v) DP3+ GOS was produced.

It is an object of the present invention to produce GOS *in situ* at high levels in milk products from the lactose already present in the milk and to provide milk products having a high concentration of *in situ* produced GOS.

### SUMMARY OF THE INVENTION

The present invention provides a milk product which comprises a total amount of at least 11 wt% of *in situ* produced DP3+ galactooligosaccharides (GOS) and wherein the *in situ* produced DP3+ GOS constitute at least 35 wt% of the total free carbohydrates, said milk product is produced in a method comprising:

(a) providing a milk substrate comprising 60% skim milk powder solution prepared rom skim milk powder containing 50% lactose and 35% protein;
(b) contacting the milk substrate with an enzyme having transgalactosylating activity for 3h at 65°C or 24h at 50, 55 or 60°C, wherein the enzyme is a Bifidobacterium bifidum β-galactosidase having the sequence of SEQ. ID. NO: 1 which was formulated with 60 w/w glucose and incubated for 69.5h at 55°C;

(c) fully or partly inactivating the enzyme, and
(d) thereby obtaining a milk product wherein at least 35wt% of the total free carbohydrates is in situ produced DP3+ galactooligosaccharides (GOS).

In a preferred embodiment, in said milk product at least 50 wt%, preferably at least 55 wt% or at least 60 wt% of the total free carbohydrates is *in situ* produced DP2+ GOS.

## DETAILED DISCLOSURE OF THE INVENTION

Milk products

Milk products comprising galactooligosaccharides (GOS) are highly desirable. GOS can be disaccharides (DP2) (except lactose which is per definition not GOS), trisaccharides (DP3), tetrasaccharides (DP4), pentasaccharides (DP5) or longer oligosaccharides. DP is short for Degree or Polymerization.

In the context of the present invention, the term "DPx+ GOS" means the sum of GOS molecules having DPx or higher DP. For example, the term "DP3+" means the sum of GOS molecules having DP3, DP4, DP5, DP6, etc.

DP3+ GOS are thus GOS having a degree of polymerization of at least 3. I.e., DP3+ GOS are trisaccharides, tetrasaccharides, pentasaccharides and longer oligosaccharides. DP3+ GOS are per definition fibre and can be claimed as such.

The dairy industry has an interest in claiming contents of GOS in their dairy products as well as contents of fibre.

High contents of *in situ* produced GOS or *in situ* produced fibre (DP3+ GOS) is of particular interest.

This invention provides a milk product which comprises a total amount of at least 11 wt% of *in situ* produced DP3+ galactooligosaccharides (GOS) and wherein the *in situ* produced DP3+ GOS constitute at least 35 wt% of the total free carbohydrates.

The free carbohydrates are the carbohydrates which are not covalently bound to proteins.

In milk products wherein the carbohydrates have not been manipulated, e.g., by enzymatic treatment, the vast majority of free carbohydrates is lactose.

The *in situ* produced GOS in the milk products have been produced in the milk substrate from the lactose present here. If a milk product is enriched by addition of preproduced GOS, such GOS are not *in situ* produced.

The milk product is obtained from milk which has not been modified in composition by addition and/or withdrawal of milk constituents except for adjustment of fat content and/or water. Obviously, in this context, an enzyme having transgalactosylating activity which has been added with the purpose of converting the lactose in the milk product to GOS is not considered a milk constituent.

The milk product is obtained from milk which has been modified in composition only by adjustment of fat content and/or water and by addition of transgalactosylating enzyme.

Adjustment of fat content and/or water may have been done by any method known in the art, e.g., by centrifugation, evaporation, condensation, ultrafiltration, nanofiltration, freeze-drying, spray-drying, reconstitution by addition of water, etc.

Not according to the claimed invention, e.g., the protein content or the lactose content may also have been slightly adjusted by, e.g., the dairy company, e.g. to obtain a milk product having a well-defined "standard" composition.

The milk product is produced from milk without altering the whey protein to casein ratio. The whey protein to casein ratio is the same as in milk.

The milk product does not comprise added lactose., Outside the subject matter of the claims, the lactose content in the milk substrate from which the milk product has been produced has only been slightly adjusted, e.g. by addition/-removal of at most 20%, preferably at most 10%, of the lactose originally present.

No milk protein has been added to or removed from the milk product. Outside the subject matter of the claims, the milk protein content in the milk product has only been slightly adjusted, e.g. by addition/removal of at most 20%, preferably at most 10%, of the milk protein originally present. This means that the overall milk protein content is largely the same as in raw milk, except for relative changes caused by removal or addition of fat or water.

Not according to the claimed invention, at most 20%, preferably at most 10%, milk protein has been added to or removed from the milk product compared to the milk protein originally present.

The milk product comprises a total amount of at least 20 wt% milk protein.

Skim milk powder is normally produced from raw milk with none or only slight adjustment of the lactose and milk protein content. Typically, skim milk powder comprises 35 wt% milk protein. Thus, a 60% skim milk solution typically comprises about 30 wt% lactose and about 21 wt% milk protein. If GOS are produced in such solution of skim milk powder from the lactose present here, such GOS are *in situ* produced GOS in the context of the present invention. If the solution of skim milk powder comprising the *in situ* produced GOS is spray dried, the milk powder obtained will be a milk product comprising *in situ* produced GOS.

Whereas if GOS are pre-produced and added as an ingredient to skim milk powder or to a solution of skim milk powder, such GOS are not *in situ* produced in the context of the present invention.

A milk product not according to the invention could also be, e.g., concentrated milk, such as condensed milk, comprising *in situ* produced GOS. Or spray-dried concentrated milk comprising *in situ* produced GOS.

The milk product claimed can be diluted (if the milk product is a liquid product such as reconstituted milk powder or concentrated milk) or solubilized (if the milk product is a milk powder) to produce a milk drink having a high relative level of *in situ* produced GOS. Or the milk product claimed can be used as an ingredient in production of, e.g., a protein bar, an infant formula or a yogurt having a high relative level of *in situ* produced GOS.

The milk product comprises 60 wt% dry matter. The milk product is prepared from reconstituted skim milk powder having 60 wt% dry matter.

The milk product is prepared from skim milk powder.

The milk product comprises 60-100 wt% dry matter.

In the milk product of the invention, the *in situ* produced DP3+ GOS constitute at least 35 wt% of the total free carbohydrate.

The wt% of DP3+ GOS of the total free carbohydrates may be determined by the method of the Examples of the present application.

In a preferred embodiment, at least 50 wt%, preferably at least 55 wt% or at least 60 wt%, of the total free carbohydrates is *in situ* produced DP2+ GOS.

The wt% of DP2+ GOS of the total free carbohydrates may be determined by the method of the Examples of the present application.

The milk product of the invention comprises a total amount of at least 11 wt% of *in situ* produced DP3+ GOS. The milk product comprises a total amount of at least 12 wt%, preferably at least 13 wt%, of *in situ* produced DP3+ GOS.

In another preferred embodiment not according to the invention, the milk product comprises 11-25 wt%, preferably 12-25 wt% or 13-25 wt%, of *in situ* produced DP3+ GOS. The milk product not according to the invention comprises 11-15 wt%, 12-15 wt% or 13-15 wt%, of *in situ* produced DP3+ GOS.

In a preferred embodiment not according to the invention, the milk product comprises a total amount of at least 12 wt%, preferably at least 15 wt% or at least 18 wt%, of *in situ* produced DP2+ GOS.

In another preferred embodiment not according to the invention, the milk product comprises 12-35 wt%, preferably 15-35 wt% or 18-35 wt%, of *in situ* produced DP2+ GOS. The milk product comprises 12-20 wt%, 15-20 wt% or 18-20 wt%, of *in situ* produced DP2+ GOS.

In a preferred embodiment not according to the invention, at most 30 wt%, preferably at most 20 wt%, of the total free carbohydrates in the milk product is lactose.

According to the invention, at most 12 wt% of the total free carbohydrates in the milk product is lactose.

As claimed the milk product is prepared from reconstituted skim milk powder and comprises a total amount of at least 11 wt%, preferably at least 12 wt% or at least 13 wt%, of *in situ* produced DP3+ GOS. In another embodiment not according to the invention, the milk product is prepared from concentrated milk, such as condensed milk, comprising a total amount of 11-20 wt%, preferably 12-20 wt% or 13-20 wt%, more preferably 11-15 wt%, 12-15 wt% or 13-15 wt%, of *in situ* produced DP3+ GOS. Such milk product preferably comprises a total amount of at most 10 wt%, preferably at most 5 wt%, lactose.

As claimed, the milk product is prepared from skim milk powder and comprises a total amount of at least 11 wt%, preferably at least 12 wt% or at least 13 wt%, of *in situ* produced DP3+ GOS. The milk product is prepared from skim milk powder and comprises a total amount of at least 15 wt%, preferably at least 18 wt% or at least 20 wt%, of *in situ* produced DP3+ GOS. Such milk product preferably comprises a total amount of at most 15 wt%, preferably at most 10 wt%, lactose.

The milk product may have been subjected to treatments known in the art such as, e.g., pasteurization, ESL treatment or UHT treatment.

Methods for producing a milk product

The milk product claimed is produced by a method comprising the following steps:

(a) providing a milk substrate comprising 60% skim milk powder solution prepared from skim milk powder containing 50% lactose and 35% protein;
(b) contacting the milk substrate with an enzyme having transgalactosylating activity as set forth in the claims,
(c) fully or partly inactivating the enzyme, and
(d) thereby obtaining a milk product wherein at least 35 wt% of the total free carbohydrates is *in situ* produced DP3+ galactooligosaccharides (GOS).

According to the claims, the milk product is produced from a milk substrate by contacting the milk substrate comprising lactose with a transgalactosylating enzyme which converts lactose in the milk substrate to GOS.

The milk substrate to be used in the method is set forth in the claims.

The term "milk", in the context of the present invention, is to be understood as the lacteal secretion obtained by milking any mammal, such as cows, sheep, goats, buffaloes or camels.

The milk substrate is obtained from milk which has not been modified in composition by addition and/or withdrawal of milk constituents except for adjustment of fat content and/or water.

In one embodiment not according to the invention, the milk substrate is obtained from milk which has been modified in composition only by adjustment of fat content and/or water.

Adjustment of fat content and/or water may have been done by any method known in the art, e.g., by centrifugation, evaporation, condensation, ultrafiltration, nanofiltration, freeze-drying, spray-drying, reconstitution by addition of water, etc.

In some embodiments not according to the invention, e.g., the protein content or the lactose content may also have been slightly adjusted by, e.g., the dairy company, e.g. to obtain a milk substrate having a well-defined "standard" composition.

The milk substrate may have been subjected to treatments known in the art such as, e.g., pasteurization, ESL treatment or UHT treatment.

The milk substrate is produced from milk without altering the whey protein to casein ratio. The whey protein to casein ratio is the same as in milk.

The milk substrate does not comprise added lactose. In another embodiment not according to the invention, the lactose content has only been slightly adjusted, e.g. by addition/removal of at most 20%, preferably at most 10%, of the lactose originally present.

In an embodiment not according to the invention, at most 20%, preferably at most 10%, lactose has been added to or removed from the milk substrate compared to the lactose originally present.

According to the invention, no milk protein has been added to or removed from the milk substrate. In another embodiment not according to the invention, the milk protein content in the milk substrate has only been slightly adjusted, e.g. by addition/removal of at most 20%, preferably at most 10%, of the milk protein originally present. According to the invention the overall milk protein content is the same as in raw milk, except for relative changes caused by removal or addition of fat or water.

In an embodiment not according to the invention, at most 20%, preferably at most 10%, milk protein has been added to or removed from the milk substrate compared to the milk protein originally present.

The milk substrate comprises 60% skim milk powder solution prepared from skim milk powder containing 50% lactose and 35% protein.

The milk substrate comprises a total amount of at least 20 wt% milk protein.

In an embodiment not according to the invention, the milk substrate is concentrated milk, such as condensed milk. According to the invention, the milk substrate is reconstituted skim milk powder as claimed.

The milk substrate comprises 60wt% dry matter. In another embodiment not according to the invention, the milk substrate is concentrated milk, such as condensed milk, which comprises at least 40 wt%, preferably at least 45 wt%, at least 50 wt%, at least 55 wt%, at least 57 wt%, or at least 60 wt% dry matter.

The milk substrate is contacted with an enzyme having transgalactosylating as set forth in the claims for for 3h at 65°C or 24h at 50, 55 or 60°C.

In an embodiment not according to the invention, the milk substrate is contacted with an enzyme having transgalactosylating activity at a temperature of 2-8°C, preferably 3-7°C, for 12 hours to 5 days, preferably 24 hours to 3 days.

According to the claims the milk substrate is contacted with an enzyme having transgalactosylating activity for 3 hours at 65° C or 24 hours at 50, 55 or 60° C.

Preferably, the enzyme having transgalactosylating activity is added at a concentration of 1-1,000 LAU(C) per g lactose, preferably 5-500 LAU(C) per g lactose, more preferably 8-150 LAU(C) per g lactose.

The activity in LAU(C) of a specific beta-galactosidase may be determined by direct measurement of glucose released from lactose. The skilled person will know how to determine such activity. Alternatively, the activity may be determined by using the activity assay described in the Methods and Examples of the present application. Here, the activity is obtained by comparing to a standard curve run with a beta-galactosidase of known activity, and the activity of the unknown sample calculated from this.

When the milk substrate has been contacted for long enough with the transgalactosylating enzyme to ensure sufficient GOS formation, the enzyme is fully or partly inactivated.

The enzyme may be inactivated by any method known in the art which would result in lowering of the enzyme activity, e.g., by heat treatment, pH or protease. It is to be understood that the inactivation may be transient. The inactivation may occur due to an inherent step in the production process of the milk product, such as pasteurization, ESL treatment, UHT treatment or spray-drying.

After the contacting of the milk substrate with the transgalactosylating enzyme, the milk product obtained may be further processed by methods known in the art, such as, e.g., centrifugation, homogenization, pasteurization, ESL treatment, UHT treatment or drying, such as spray-drying or freeze-drying.

In the milk product obtained by the method of the invention, at least 35 wt% of the total free carbohydrates must be *in situ* produced DP3+ GOS obtained as a result of the contacting of the milk substrate with the transgalactosylating enzyme.

The milk product produced by the method of the invention comprises a total amount of at least 11 wt% of *in situ* produced DP3+ GOS.

Milk products comprising 11-20 wt%, preferably 12-20 wt% or 13-20 wt%, more preferably 11-15 wt%, 12-15 wt% or 13-15 wt%, of *in situ* produced DP3+ GOS are disclosed are however not according to the present invention.

The milk product comprises a total amount of at least 12 wt%, preferably at least 15 wt% or at least 18 wt%, of *in situ* produced DP2+ GOS.

Milk products comprising 12-25 wt%, preferably 15-25 wt% or 18-25 wt%, more preferably 12-20 wt%, 15-20 wt% or 18-20 wt%, of *in situ* produced DP2+ GOS are disclosed are however not according to the present invention.

At most 12 wt%, of the total free carbohydrates in the milk product obtained by the method is lactose.

The milk product obtained by the method according to the claims comprises a total amount of at least 11 wt%, preferably at least 12 wt% or at least 13 wt%, of *in situ* produced DP3+ GOS. Such milk product preferably comprises a total amount of at most 10 wt%, preferably at most 5 wt%, lactose.

In one embodiment not according to the invention, the method further comprises a step (e): (e) drying, preferably spray-drying, the milk product of step (d) to obtain a milk powder, such as a skim milk powder.

The milk product of step (d) or the milk powder of step (e) may be used in the production of, e.g., a protein bar, a milk drink, an infant formula or a yoghurt.

In one embodiment outside the subject matter of the claims, the method is a method for producing a fermented milk product which comprises:

(a) providing a milk substrate comprising at least 20% (w/v) lactose,
(b) contacting the milk substrate with an enzyme having transgalactosylating activity for at least 45 minutes,
(c) fermenting the milk substrate, preferably by incubating with a bacterial culture, before, during and/or after step (b),
(d) inactivating the enzyme before or after step (c), and
(e) thereby obtaining a fermented milk product wherein at least 35 wt% of the total free carbohydrates is *in situ* produced DP3+ galactooligosaccharides (GOS).

Enzyme having transgalactosylating activity

The milk substrate is contacted with an enzyme having transgalactosylating activity.

Beta-galactosidases from glycoside hydrolase family 2 (GH2) are exo-acting enzymes, which hydrolyse terminal non-reducing beta-D-galactose residues in beta-D-galactosides, e.g. lactose is hydrolysed to galactose and glucose. They belong to the enzyme class EC 3.2.1.23 with the official name beta-D-galactoside galactohydrolase. A common name used for this enzyme is lactase, as lactose is the common industrial substrate. Besides hydrolysing this enzyme class is also able to transfer galactose to other sugars and thereby make galacto-oligosaccharides (GOS). The different GH2 enzyme have various preferences for hydrolytic or beta-galactosidase activity and transgalactosylase activity and the preference can be expressed in terms of their GOS production ability, such as by the ratio of transgalactosylating to hydrolysing activity.

In the present context, the term "beta-galactosidase" means any glycoside hydrolase having the ability to hydrolyse the disaccharide lactose into its constituent galactose and glucose monomers. Enzymes assigned to subclass EC 3.2.1.108, also called lactases, are also considered a beta-galactosidase in the context of the present invention. In the context of the invention, the lactose hydrolysing activity of the beta-galactosidase may be referred to as its lactase activity or its beta-galactosidase activity.

In the context of the present invention, the enzyme having transgalactosylating activity belongs to the enzyme class EC 3.2.1.23.

In certain applications, combinations of polypeptides having predominantly transgalactosylating activity and predominantly hydrolysing activity may be contemplated. This may be especially useful when there is a desire to reduce residual lactose after treatment with the polypeptide having beta-galactosidase activity, for example at low lactose levels.

When considering the reaction of the enzyme in e.g. milk, carbohydrates are initially present in the form of lactose, a disaccharide composed of galactose and glucose that is found in milk. In the formation of GOS, successive galactose molecules are added to lactose, and then after prolonged incubation a mixture of the various carbohydrates is present (glucose, galactose and ~30 different di- and polysaccharides).

The term "disaccharide" as used herein means two monosaccharide units bound together by a covalent bond known as a glycosidic linkage formed via a dehydration reaction, resulting in the loss of a hydrogen atom from one monosaccharide and a hydroxyl group from the other.

As used herein, the terms "enzyme having transgalactosylating activity", "transgalactosylating enzyme" and "transgalactosylase" are used interchangeably to mean an enzyme that is able to transfer galactose to the hydroxyl groups of D-galactose (Gal) or D-glucose (Glc) whereby galactooligosaccharides are produced. According to the description , transgalactosylase activity is identified by reaction of the enzyme on lactose in which the amount of galactose generated is less than the amount of glucose generated at a given time.

More particularly, the transgalactosylase activity (or preference for an enzyme to either hydrolyze lactose or to produce GOS) can be evaluated as the amount of glucose minus galactose generated at any given time during reaction or by direct quantification of GOS generated during the reaction. This measurement may be performed by one of several ways including the methods shown in the Examples herein.

When evaluating the transgalactosylating activity versus hydrolysing activity of an enzyme, the hydrolysing activity may be measured as the concentration of galactose ([Gal]) generated at any time point during the reaction.

The transgalactosylating activity, i.e. the transfer of a galactose moiety to a molecule other than water, may be measured as ([Glc] - [Gal]) generated at any given time.

Thus, the ratio of transgalactosylating to hydrolysing activity in the context of the present invention is determined as ([Glc] - [Gal])/[Gal].

Enzymes having transgalactosylating activity may be of animal, of plant or of microbial origin, such as from a filamentous fungus or yeast, or from a bacterium, e.g.

*Agaricus*, e.g. *A. bisporus; Ascovaginospora; Aspergillus*, e.g. *A. niger, A. awamori, A. foetidus, A. japonicus, A. oryzae; Candida; Chaetomium; Chaetotomastia; Dictyostelium*, e.g. *D. discoideum; Kluveromyces*, e.g. *K. fragilis, K. lactis; Mucor*, e.g. *M. javanicus, M. mucedo, M. subtilissimus; Neurospora*, e.g. *N. crassa; Rhizomucor*, e.g. *R. pusillus; Rhizopus*, e.g. *R. arrhizus, R. japonicus, R. stolonifer; Sclerotinia*, e.g. *S. libertiana; Torula; Torulopsis; Trichophyton*, e.g. *T. rubrum; Whetzelinia*, e.g. *W. sclerotiorum; Bacillus*, e.g. *B. sp. B. coagulans, B. circulans, B. megaterium, B. novalis, B. subtilis, B. pumilus, B. stearothermophilus, B. thuringiensis; Bifidobacterium*, e.g. *B. animalis, B. bifidum, B. breve, B. infantis, B. lactis, B. longum; Chryseobacterium; Citrobacter*, e.g. *C. freundii; Clostridium*, e.g. *C. perfringens; Diplodia*, e.g. *D. gossypina; Enterobacter*, e.g. *E. aerogenes, E. cloacae Edwardsiella, E. tarda; Erwinia*, e.g. *E. herbicola; Escherichia*, e.g. *E. coli; Klebsiella*, e.g. *K. pneumoniae; Miriococcum; Myrothesium; Mucor, Neurospora*, e.g. *N. crassa; Proteus*, e.g. *P. vulgaris; Providencia*, e.g. *P. stuartii; Pycnoporus*, e.g. *Pycnoporus cinnabarinus, Pycnoporus sanguineus; Ruminococcus*, e.g. *R. torques; Salmonella*, e.g. *S. typhimurium; Serratia*, e.g. *S. liquefasciens, S. marcescens; Shigella*, e.g. *S. flexneri; Streptomyces*, e.g. *S. antibioticus, S. castaneoglobisporus, S. violeceoruber; Trametes; Trichoderma*, e.g. *T. reesei, T. viride; Yersinia*, e.g. *Y. enterocolitica.*

According to the claims, the enzyme is a beta-galactosidase from *Bifidobacterium bifidum.*

In an embodiment outside the scope of the claims, the enzyme is a beta-galactosidase from a bacterium, e.g. from the family Bacillaceae, such as from the genus *Bacillus,* such as from a strain of *B. sp., B. coagulans, B. circulans, B. megaterium, B. novalis, B. subtilis, B. pumilus, B. stearothermophilus, B. thuringiensis; Bifidobacterium,* e.g. *B. animalis, B. bifidum, B. breve, B. infantis, B. lactis, B. longum.* In a more preferred embodiment, the enzyme is a beta-galactosidase from *Bacillus circulans* or *Bacillus infantis.*

According to the invention, the enzyme has SEQ ID NO: 1.

In an embodiment not according to the invention, the enzyme having transgalactosylating activity to be used in the method comprises an amino acid sequence which is at least 50% identical to amino acids 28-1931 of SEQ ID NO: 2, or a fragment thereof having transgalactosylating activity. In an embodiment not according to the invention, the enzyme comprises an amino acid sequence which is at least 60%, such as at least 70%, at least 80%, at least 90%, at least 95% or at least 98% identical to amino acids 28-1931 of SEQ ID NO: 2.

In another embodiment not according to the invention, the enzyme having transgalactosylating activity to be used in the method has an amino acid sequence which is at least 50% identical to amino acids 28-1331 of SEQ ID NO: 3, or a fragment thereof having transgalactosylating activity. Preferably, the polypeptide has an amino acid sequence which is at least 60%, such as at least 70%, at least 80%, at least 90%, at least 95% or at least 98% identical to amino acids 28-1331 of SEQ ID NO: 3.

In another embodiment not according to the invention, the enzyme having transgalactosylating activity to be used in the method has an amino acid sequence which is at least 50% identical to SEQ ID NO: 4, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 4.

In another embodiment not according to the invention, the enzyme having transgalactosylating activity to be used in the method has an amino acid sequence which is at least 50% identical to SEQ ID NO: 5, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 5.

In another embodiment not according to the invention, the enzyme having transgalactosylating activity to be used in the method has an amino acid sequence which is at least 50% identical to SEQ ID NO: 6, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 6.

In another embodiment not according to the invention, the enzyme having transgalactosylating activity to be used in the method has an amino acid sequence which is at least 50% identical to SEQ ID NO: 7, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 7.

In another embodiment not according to the invention, the enzyme having transgalactosylating activity to be used in the method has an amino acid sequence which is at least 50% identical to SEQ ID NO: 8, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 8.

In another embodiment not according to the invention, the enzyme having transgalactosylating activity to be used in the method has an amino acid sequence which is at least 50% identical to SEQ ID NO: 9, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 9.

In another embodiment not according to the invention, the enzyme having transgalactosylating activity to be used in the method has an amino acid sequence which is at least 50% identical to SEQ ID NO: 10, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 10.

In another embodiment not according to the invention, the enzyme having transgalactosylating activity to be used in the method has an amino acid sequence which is at least 50% identical to SEQ ID NO: 11, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 11.

In another embodiment not according to the invention, the enzyme having transgalactosylating activity to be used in the method has an amino acid sequence which is at least 50% identical to SEQ ID NO: 12, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 12.

In another embodiment not according to the invention, the enzyme having transgalactosylating activity to be used in the method has an amino acid sequence which is at least 50% identical to SEQ ID NO: 13, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 13.

In another embodiment not according to the invention, the enzyme having transgalactosylating activity to be used in the method has an amino acid sequence which is at least 50% identical to SEQ ID NO: 14, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 14.

In another embodiment not according to the invention, the enzyme having transgalactosylating activity to be used in the method has an amino acid sequence which is at least 50% identical to SEQ ID NO: 15, or a fragment thereof having transgalactosylating activity. Preferably, the polypeptide has an amino acid sequence which is at least 60%, such as at least 70%, at least 80%, at least 90%, at least 95% or at least 98% identical to SEQ ID NO: 15.

The sequence identity between two amino acid sequences may be determined as the output of "longest identity" using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 6.6.0 or later. The parameters used are a gap open penalty of 10, a gap extension penalty of 0.5, and the EBLOSUM62 (EMBOSS version of BLOSUM62) substitution matrix. In order for the Needle program to report the longest identity, the -nobrief option must be specified in the command line. The output of Needle labeled "longest identity" is calculated as follows:

$$\text{(Identical Residues x 100)/(Length of Alignment – Total Number of Gaps in Alignment)}$$

The enzyme having transgalactosylating activity may be extracellular. It may have a signal sequence at their N-terminus, which is cleaved off during secretion.

The enzyme having transgalactosylating activity may be derived from any of the sources mentioned herein. The term "derived" means in this context that the enzyme may have been isolated from an organism where it is present natively, i.e. the identity of the amino acid sequence of the enzyme are identical to a native polypeptide. The term "derived" also means that the enzyme may have been produced recombinantly in a host organism, the recombinantly produced enzyme having either an identity identical to a native enzyme or having a modified amino acid sequence, e.g. having one or more amino acids which are deleted, inserted and/or substituted, i.e. a recombinantly produced enzyme which is a mutant and/or a fragment of a native amino acid sequence. Within the meaning of a native enzyme are included natural variants. Furthermore, the term "derived" includes enzymes produced synthetically by, e.g., peptide synthesis. The term "derived" also encompasses enzymes which have been modified e.g. by glycosylation, phosphorylation etc., whether *in vivo* or *in vitro*. With respect to recombinantly produced enzymes the term "derived from" refers to the identity of the enzyme and not the identity of the host organism in which it is produced recombinantly.

The enzyme having transgalactosylating activity may be obtained from a microorganism by use of any suitable technique. For instance, an enzyme preparation may be obtained by fermentation of a suitable microorganism and subsequent isolation of a lactase preparation from the resulting fermented broth or microorganism by methods known in the art. The enzyme having transgalactosylating activity may also be obtained by use of recombinant DNA techniques. Such method normally comprises cultivation of a host cell transformed with a recombinant DNA vector comprising a DNA sequence encoding the lactase in question and the DNA sequence being operationally linked with an appropriate expression signal such that it is capable of expressing the enzyme in a culture medium under conditions permitting the expression of the enzyme and recovering the enzyme from the culture. **The** DNA sequence may also be incorporated into the genome of the host cell. **The** DNA sequence may be of genomic, cDNA or synthetic origin or any combinations of these, and may be isolated or synthesized in accordance with methods known in the art.

**The** enzyme having transgalactosylating activity may be purified. **The** term "purified" as used herein covers beta-galactosidase enzyme protein essentially free from insoluble components from the production organism. The term "purified" also covers beta-galactosidase enzyme protein essentially free from insoluble components from the native organism from which it is obtained. Preferably, it is also separated from some of the soluble components of the organism and culture medium from which it is derived. More preferably, it is separated by one or more of the unit operations: filtration, precipitation, or chromatography.

Accordingly, the enzyme having transgalactosylating activity may be purified, viz. only minor amounts of other proteins being present. The expression "other proteins" relate in particular to other enzymes. The term "purified" as used herein also refers to removal of other components, particularly other proteins and most particularly other enzymes present in the cell of origin of the beta-galactosidase. The enzyme having transgalactosylating activity may be "substantially pure", i.e. free from other components from the organism in which it is produced, i.e., e.g., a host organism for recombinantly produced beta-galactosidase. Preferably, the enzyme having transgalactosylating activity is an at least 40% (w/w) pure enzyme protein preparation, more preferably at least 50%, 60%, 70%, 80% or even at least 90% pure.

The term enzyme having transgalactosylating activity includes whatever auxiliary compounds may be necessary for the enzyme's catalytic activity, such as, e.g., an appropriate acceptor or cofactor, which may or may not be naturally present in the reaction system.

The enzyme may be in any form suited for the use in question, such as, e.g., in the form of a dry powder or granulate, a non-dusting granulate, a liquid, a stabilized liquid, or a protected enzyme.

Glycation

Despite the dominant hydrolytic properties of certain beta-galactosidase or lactase enzymes, these enzymes can be converted to have higher transferring properties, e.g., by subjecting the enzymes to a pre-incubation. The pre-incubation thus results in a more robust GOS-producing enzyme due to its higher transferring abilities (transgalactosylase activity).

Without wishing to be bound by theory, it is believed that these incubation conditions result in glycation of the beta-galactosidase, which results in increased transferring properties. With covalent attachment of the sugar moiety, the beta-galactosidase is converted from a hydrolysing to a transferring enzyme having transgalactosylase activity.

According to the claims, the enzyme having transgalactosylating activity has SEQ.1 and was formulated with 60 w/w glucose and incubated for 69.5h at 55°C. "Glycation" as used herein refers to the covalent attachment of a carbohydrate to a protein.

Carbohydrate attachment may be via a side chain of, e.g., arginine, lysine, or N-terminal of the enzyme. Preferably, the carbohydrate attachment is via a side chain of arginine or lysine.

Glycation is sometimes referred to as (non-enzymatic) glycosylation. In the context of the present invention, glycosylation and glycation are used interchangeably and glycosylation can be non-enzymatic.

The enzyme having transgalactosylating activity has been modified by glycation of at least 1, 2, 3, 4, 5, 6, 7, 8, 9, 10, 15, 20, 25, 30, 35, 40, 45, 50, 55, 60 residues of the enzyme.

The enzyme having transgalactosylating activity has been modified by glycation of at least 1, 2, 3, 4, 5, 6, 7, 8, 9, 10, 15, 20, 25, 30, 35, 40, 45, 50, 55, 60 lysine and/or arginine residues of the enzyme.

The enzyme having transgalactosylating activity has been modified by glycation of at least 1%, 2%, 3%, 4%, 5%, 10%, 15%, 20%, 25%, 30%, 35%, 40%, 45% or 50% of the lysine and/or arginine residues of the enzyme.

The enzyme having transgalactosylating activity has been modified by glycation of at least 1, 2, 3, 4, 5, 6, 7, 8, 9, 10, 15, 20, 25, 30, 35, 40, 45, 50, 55, 60 lysine and/or arginine residues of the enzyme.

Incubation under suitable conditions as detailed below results in the glycation of some, substantially all or, or even all of the surface residues of lysine and/or arginine. Again without wishing to be bound by theory, it is believed that some, substantially all, or even all of the glycated residues are located towards the C-terminal end of the enzyme having transgalactosylating activity.

According to the claims, the sugar used for glycation is glucose.

According to the claims, the enzyme having transgalactosylating activity has been formulated with 60 w/w glucose and incubated for 69.5 hours at 55°C.

The presence of a reducing sugar can be detected by many well-known tests including the use of Benedict's reagent and/or Tollen's reagent.

## EXAMPLES

## MATERIALS AND METHODS

*Activity Assay (LAU(C))*

Principle:

Lactase hydrolyzes lactose to form α-D-glucose. The α-D-glucose is phosphorylated by ATP, in a reaction catalyzed by hexokinase. The glucose-6-phosphate formed is oxidized to 6-phosphogluconate by glucose-6-phosphate dehydrogenase. Concommittant with this reaction an equimolar amount of NAD+ is reduced to NADH with a resulting increase in absorbance at 340 nm.

Reagents:

15% (w/v) Brij L23: Weigh out 508.0 ± 0.4g of Brij® L23 (Sigma B4184) into a beaker. Add approx. 300mL ultrapure water and stir. Transfer the Brij® L23 quantitatively to a 1 L volumetric flask. Fill to the mark with ultrapure water. Stir until homogenous. Storability: 2 months in refrigerator.

Colour reagent: (Glucose reagent kit (GHK) (0.1 M Tris, 2.1 mM ATP, 2.1 mM NAD, 4 mM Mg2+, <0.1% NaN3, 4 mMMg2+, >7.5 kU/L hexokinase, > 7.5 kU/L G-6-P-DH, pH 7.8)): Open a vial of Glucose (HK) Reagent A, Thermo Fisher Scientific (Art. no.: 981304 or 981779) and a vial of Glucose (HK) Reagent B, Thermo Fisher Scientific (Art. no.: 981304 or 981779). Pour 1 vial of reagent B into 1 vial of reagent A. Put on the cap. Mix well by slowly and gently turning up and down the vial 10-15 times. Use the whole mixture in reagent A vial, or pour needed amount into an appropriate container. Storability: 1 month in refrigerator. Dissolution buffer/dilution buffer (0.01 M Citric acid monohydrate, 0.0225% (w/v) Brij® L23, 1 mM MgSO4, 7H2O, pH 4.5): Weigh out 21.0 ± 0.1 g of Citric acid monohydrate (Cas. No. 5949-29-1) and transfer quantitative to a 10 L volumetric flask. Weigh out 2.46 ± 0.01 g of MgSO4, 7H2O (Cas. no. 10034-99-8) and transfer quantitative to the volumetric flask. Add approximately 9 L of demineralized water and stir until completely dissolved. Add 15 mL of 15% (w/v) Brij L23 to the volumetric flask and stir. Add approximately 35 mL of 4 M NaOH (Cas. No. 1310-73-2) and stir. Adjust pH to 4.50 ± 0.05 using e.g. 4 M NaOH or e.g. HCl as appropriate. Fill to the mark with demineralized water and stir. Storability: 13 days at room temperature.

Substrate (31.6 % w/w lactose monohydrate, 0.01M citric monohydrate, 0.0225 (w/v) Brij L23, 1 mM MgSO4, 7H2O): Weigh out 7.9 ± 0.2 g of Lactose monohydrate (Cas. No. 10039-26-6) directly into a beaker. Dissolve to a total volume of 25.0 ± 0.1 g of dissolution buffer. Heat up and stir until fully dissolved with no boiling of the substrate. Storability: 6 hours at room temperature.

Standard: Enzyme standard with identified LAU(C)/g (available from Novozymes A/S, Denmark) is used as standard, diluted in dissolution buffer in the range from 0.197-0.7880 LAU(C)/mL.

Procedure:

1. 50 uL of substrate is incubated for 540 seconds at 50°C. Blank (50 uL of dissolution buffer) is subtracted out.
2. 25 uL sample in dissolution buffer is added.
3. The reaction is incubated for 1800 seconds followed by addition of 160 uL colour reagent.
4. After 300 seconds, the absorbance is measured at 340 nm.

Calculation of Enzyme Activity:

The enzyme activity of the diluted sample is read from the standard curve. Calculation of activity of a sample in LAU(C)/g is as stated in the formula:

$$Activity\ Unit\,/\,g\ =\ \frac{S \cdot V \cdot F}{W}$$

S = Reading from the standard curve in LAU(C)/mL
V = Volume of the measuring flask used in mL
F = Dilution factor for second dilution
W = Weight of sample in g

## EXAMPLE 1

### Sample:

*Bifidobacterium bifidum* β-galactosidase having the sequence shown as SEQ ID NO: 1 has been expressed in *Bacillus licheniformis* and concentrated using UF (cut-off 10 kDa) and finally formulated with glucose 60% (w/w) and incubated for 69.5 h at 55°C and then stored at -20°C. Concentration of this sample is 5300 LAU(C)/g.

### GOS production in regular milk.

#### 5°C for 24 h (Results are shown in Table 1).- **not according to the invention**

Two ml semi-skim milk (Arla, purchased in a local Danish supermarket, 4.7 g lactose and 3,5 g protein per 100 g) were transferred into a 5 ml Eppendorf tube (double determinations for each dose including control). Then 20 μl of enzyme dilution was added (final LAU(C)/I can be seen in Table 1) and mixed followed by an incubation at 5°C for 24 h. After incubation, 10 μl concentrated acetic acid was added to each sample and the solutions were heated to 90°C for 5 min. After inactivation, the samples were centrifuged at 14,000 rpm for 5 min at room temperature. One ml supernatant was transferred to another tube and kept frozen until analyzed by HPLC.

#### 60°C for 3 h (Results are shown in Table 3) - **not according to the invention**

Two ml semi-skim milk (Arla, purchased in a local Danish supermarket, 4.7 g lactose and 3.5 g protein per 100 g) were transferred into a 2 ml Eppendorf tube (double determinations for each dose including control). Then 20 μl of enzyme dilution was added (final LAU(C)/I can be seen in Table 3) and mixed followed by an incubation at 60°C for 3h. After incubation, 10 μl concentrated acetic acid was added to each sample and the solutions were heated to 90°C for 5 min. After inactivation, the samples were centrifuged at 14,000 rpm for 5 min at room temperature. One ml supernatant was transferred to another tube and kept frozen until analyzed by HPLC.

### GOS production in 60% skim milk powder solution (SMP)

#### 5°C for 22.5 h (Results are shown in Table 2) - **not according to the invention**

12 g skim milk powder (containing 50% lactose and 35% protein) and 8 ml sample solution (final LAU(C)/kg 60% SMP can be seen in Table 2) was added to a 50 ml tube and mixed and incubated for 22.5 h at 5°C. Then 20 ml 3% HAc was added and mixed with a spatula until a homogenous solution and then placed on a rotator at room temp. for 10 min. Then a centrifugation at 7850 rpm for 10 min was performed and 200 μl supernatant was transferred to a 2 ml Eppendorf tube containing 1 ml water and heated to 90°C for 5 min and centrifuged at 14,000 rpm for 5 min at room temp. and supernatant was kept frozen until use for the two analytic HPLC tests.

#### 50, 55 and 60°C for 24 h and 65°C for 3 h (Results are shown in Table 4)

12 g skim milk powder (containing 50% lactose and 35% protein) and 8 ml sample solution (final LAU(C)/kg 60% SMP can be seen in Table 4) was added to a 50 ml tube and mixed and incubated for 24 h at 50, 55 or 60°C or for 3 h at 65°C. Then 20 ml 3% HAc was added and mixed with a spatula until a homogenous solution and then placed on a rotator at room temp for 10 min. Then a centrifugation at 7850 rpm for 10 min was performed and 200 μl supernatant was transferred to a 2 ml Eppendorf tube containing 1 ml water and heated to 90°C for 5 min and centrifuged at 14,000 rpm for 5 min at room temp. and supernatant was kept frozen until use for the two analytic HPLC tests.

## Determination of ratio of lactose (Lac) and GOS di-saccharides (DP2-GOS)

High-Performance Anion-Exchange Chromatography with Pulsed Amperometric Detection (HPAEC-PAD) using a PA1 column for quantitative determination of galactose (Gal), glucose (Glc), lactose (Lac) and DP2-GOS's is performed as follows.

50 $\mu$l sample is mixed together with 500 $\mu$l Milli Q water + 10 $\mu$l Carrez I solution in a 5 ml Eppendorf tube, and then mixed with 10 $\mu$l Carrez II solution. Then 4.43 ml milli Q water is added and centrifugated at 14,000 rpm for 5 min at room temperature. One ml supernatant is mixed with 4 ml Milli-Q water and transferred to spin-x filter tubes and centrifugated for 5 min at 6,000 rpm and applied on a PA1 column. Quantitative determination of Lac, b-1-6-Gal-Gal, b-1-6-Gal-Glc, b-1-3-Gal-Gal, b-1-4-Gal-Gal and b-1-3-Gal-Glc (+ b-1-2-Gal-Glc) was made so a ratio of GOS di-saccharides (DP2G) and lactose could be determined.

This DP2G/(Lac+DP2G) ratio is then used to calculate the amount of DP2G in the DP2 peak determined by the Dionex HPLC system ICS-5000 RI method (see below) and total GOS can then be calculated by the sum of DP3+ and DP2G.

Likewise, the Lac/(Lac+DP2G) ratio (= 1 minus DP2G/(Lac+DP2G)) can be used to calculate the amount of lactose in the DP2 peak determined below.

## Quantitative determination of Glc, Gal, DP2, DP3, DP4 and DP5+

Dionex HPLC system ICS-5000 RI (Aminex-HPX-87H Ion Exclusion Column) is used for quantitative determination of Gal, Glc, DP2, DP3, DP4 and DP5+. Undiluted sample is transferred to spin-x filter tubes and centrifugated for 5 min at 6,000 rpm and applied to an Aminex-HPX-87H column. A control sample without enzyme addition was incubated under the same conditions as enzyme treated samples and used to calculate the contribution of Gal, Glc and DP3, DP4 and DP5+ from the enzyme treatment.

The numbers shown in the tables are based on the quantitative determination of Gal, Glc, DP3, DP4 and DP5+ minus the quantitative amount of Gal, Glc, DP3, DP4 and DP5+ estimated in the control (without enzyme) sample.

Quantitative DP3+ is determined as DP3 + DP4 + DP5+.

## Quantitative determination of GOS di-saccharides (DP2-GOS) and lactose (Lac)

Quantitative lactose (Lac) = DP2 multiplied by the Lac/(Lac+DP2G) ratio determined above.

Quantitative DP2G = DP2 multiplied by the DP2G/(Lac+DP2G) ratio determined above.

Finally, quantitative GOS = DP2G plus DP3+.

Results

Table 1. GOS production in regular milk at 5°C for 24 h

| | Glucose | Glucose | Galactose | Galactose | Lac | Lac | DP3 + | DP3 + | GOS | GOS | (Glc-Gal)/Gal |
|---|---|---|---|---|---|---|---|---|---|---|---|
| | avr | avrdev | avr | avrdev | avr | avrdev | avr | avrdev | avr | avrdev | |
| LAU (C)/g lactose | % | % | % | % | % | % | % | % | % | % | |
| 31 | 8.1 | 0.16 | 3.3 | 0.31 | 70. 2 | 0.26 | 11. 4 | 0.07 | 18. 4 | 0.21 | 1.5 |
| 62 | 14.8 | 0.01 | 5.9 | 0.03 | 49. 2 | 0.07 | 18. 9 | 0.09 | 30. 1 | 0.03 | 1.5 |
| 93 | 19.9 | 0.13 | 7.8 | 0.05 | 34. 3 | 0.29 | 23. 8 | 0.09 | 38. 0 | 0.11 | 1.6 |
| 124 | 25.1 | 0.01 | 10.0 | 0.00 | 19. 8 | 0.02 | 27. 5 | 0.00 | 45. 1 | 0.02 | 1.5 |
| 155 | 27.6 | 0.06 | 11.4 | 0.02 | 13. 5 | 0.49 | 28. 0 | 0.03 | 47. 4 | 0.57 | 1.4 |

(continued)

| | Glucose | Glucose | Galactose | Galactose | Lac | Lac | DP3 + | DP3 + | GOS | GOS | (Glc-Gal)/ Gal |
|---|---|---|---|---|---|---|---|---|---|---|---|
| | avr | avrdev | avr | avrdev | avr | avrdev | avr | avrdev | avr | avrdev | |
| LAU (C)/g lactose | % | % | % | % | % | % | % | % | % | % | |
| 186 | 29.4 | 0.01 | 12.8 | 0.01 | 10. 4 | 0.03 | 27. 3 | 0.06 | 47. 4 | 0.01 | 1.3 |
| 217 | 30.9 | 0.07 | 14.1 | 0.06 | 8.0 | 0.08 | 25. 7 | 0.11 | 47. 1 | 0.05 | 1.2 |
| 249 | 31.9 | 0.09 | 15.2 | 0.08 | 6.7 | 0.05 | 24. 0 | 0.21 | 46. 2 | 0.11 | 1.1 |
| 280 | 33.3 | 0.05 | 17.0 | 0.12 | 5.6 | 0.04 | 20. 9 | 0.20 | 44. 1 | 0.13 | 1.0 |
| 311 | 34.2 | 0.07 | 18.0 | 0.09 | 5.0 | 0.03 | 19. 0 | 0.18 | 42. 8 | 0.13 | 0.9 |
| 388 | 36.2 | 0.11 | 20.8 | 0.06 | 4.0 | 0.01 | 14. 2 | 0.23 | 39. 1 | 0.18 | 0.7 |
| 466 | 38.0 | 0.03 | 23.4 | 0.02 | 3.1 | 0.01 | 10. 5 | 0.07 | 35. 5 | 0.06 | 0.6 |

Table 2. GOS production in 60% SMP at 5°C for 22.5 h

| | Glc | Gal | Lac | DP3+ | GOS | (Glc-Gal)/Glc |
|---|---|---|---|---|---|---|
| LAU(C)/g Lac | % | % | % | % | % | ratio |
| 7 | 7.0 | 0.7 | 75.5 | 14.6 | 16.8 | 8.9 |
| 13 | 9.4 | 1.0 | 67.4 | 19.0 | 22.2 | 8.7 |
| 27 | 13.8 | 1.5 | 52.9 | 26.7 | 31.7 | 8.3 |
| 53 | 18.9 | 2.1 | 35.9 | 35.9 | 43.1 | 8.0 |
| 106 | 22.6 | 3.1 | 23.4 | 38.7 | 50.9 | 6.3 |
| 212 | 23.5 | 4.7 | 14.3 | 34.1 | 57.5 | 4.0 |

To claim fibre in a dairy product, a high DP3+ (DP3+ is DP3 and higher DP which is fibre per definition) is requested. If starting substrate is regular milk, seen in Table 1, a maximum of 28.0 % DP3+ can be obtained, whereas having 60% SMP a maximum of 38.7% can be obtained, see Table 2. The higher DP3+ yield obtained by the 60% SMP application enables, besides fibre claim, also lower calories of the final milk product as DP3+ have 2 kcal/g whereas Glc, Gal and DP2G have 4 kcal/g as described by EU regulatory: ANNEX XIV, Regulation 1169/2011 on Food Information to Consumers.

Table 3. GOS production in regular milk at 60°C for 3 h

| | Glucose | Glucose | Galactose | Galactose | Lac | Lac | DP3 + | DP3 + | GO S | GOS | (Glc-Gal)/ Gal |
|---|---|---|---|---|---|---|---|---|---|---|---|
| | Avr | AvrD ev | Avr | AvrDev | Avr | AvrD ev | Avr | AvrD ev | Avr | AvrD ev | |
| LAU (C)/ g lactose | % | % | % | % | % | % | % | % | % | % | |
| 16 | 2.0 | 0.17 | 0.5 | 0.09 | 92.6 | 0.84 | 3.8 | 0.43 | 4.9 | 0.58 | 3.0 |
| 31 | 4.0 | 0.24 | 1.0 | 0.09 | 83.9 | 0.87 | 7.1 | 0.51 | 11. 0 | 0.53 | 2.9 |
| 62 | 8.3 | 0.23 | 2.3 | 0.11 | 68.1 | 0.75 | 14. 8 | 0.28 | 21. 3 | 0.41 | 2.6 |
| 93 | 11.5 | 0.11 | 3.4 | 0.06 | 57.1 | 0.70 | 19. 3 | 0.25 | 28. 0 | 0.54 | 2.4 |

(continued)

| | Glucose | Glucose | Galactose | Galactose | Lac | Lac | DP3+ | DP3+ | GO S | GOS | (Glc-Gal)/ Gal |
|---|---|---|---|---|---|---|---|---|---|---|---|
| | Avr | AvrD ev | Avr | AvrDev | Avr | AvrD ev | Avr | AvrD ev | Avr | AvrD ev | |
| LAU (C)/ g lactose | % | % | % | % | % | % | % | % | % | % | |
| 124 | 14.3 | 0.35 | 4.1 | 0.10 | 48.4 | 0.97 | 22. 7 | 0.29 | 33. 2 | 0.51 | 2.5 |
| 155 | 16.5 | 0.39 | 4.8 | 0.13 | 42.0 | 1.23 | 25. 1 | 0.34 | 36. 7 | 0.71 | 2.5 |
| 311 | 26.2 | 0.28 | 8.5 | 0.11 | 17.5 | 0.53 | 31. 3 | 0.07 | 47. 8 | 0.15 | 2.1 |

Table 4. GOS production in 60% SMP at 50, 55 and 60°C for 24 h and 65°C for 3 h

| incubation temp | Time | Dose | Glucose | Galactose | Lac | DP3+ | GOS | (Glc-Gal)/Ga l |
|---|---|---|---|---|---|---|---|---|
| °C | h | LAU(C)/g Lac | % | % | % | % | % | |
| 50 | 24 | 13 | 23.2 | 4.1 | 10.4 | 43.3 | 62.3 | 4.6 |
| 55 | 24 | 13 | 23.0 | 4.3 | 10.1 | 43.2 | 62.5 | 4.3 |
| 60 | 24 | 13 | 22.3 | 3.8 | 10.6 | 44.4 | 63.4 | 4.9 |
| 65 | 3 | 53 | 22.7 | 2.6 | 10.8 | 44.8 | 63.9 | 7.7 |

Table 3 shows GOS made in regular milk at 60°C for 3 h with increasing enzyme dose (LAU(C)/kg, column 1). At the highest enzyme dose, a DP3+ of 31.3% was obtained which is 3.3% higher compared to the max. DP3+ obtained at 5°C (see Table 1). The same relative increase in DP3+ is seen at 60% SMP at high temperatures (50-65°C, table 4) compared to 60% SMP at 5°C (table 2). Thus, both in regular milk and 60% SMP a relative increase of DP3+ can be obtained at higher temp.

Table 4 shows that it is possible to obtain reconstituted skim milk powder (60%) wherein more than 44 wt% of the total free carbohydrates is *in situ* produced DP3+ GOS. The skim milk powder used contained 50% lactose. Thus, the total amount of *in situ* produced DP3+ GOS in the reconstituted skim milk powder is more than 13 wt% (44% x 60% x 50%). If the reconstituted skim milk powder obtained were dried, e.g. by spray-drying, the skim milk powder obtained would comprise more than 22 wt% of *in situ* produced DP3+ GOS (44% x 50%).

The invention described and claimed herein is not to be limited in scope by the specific aspects herein disclosed, since these aspects are intended as illustrations of several aspects of the invention.

## Claims

1. A milk product

which comprises a total amount of at least 11 wt% of *in situ* produced DP3+ galactooligosaccharides (GOS) and wherein the *in situ* produced DP3+ GOS constitute at least 35 wt% of the total free carbohydrates,

said milk product is produced in a method comprising:

(a) providing a milk substrate comprising 60% skim milk powder solution prepared from skim milk powder containing 50% lactose and 35% protein;
(b) contacting the milk substrate with an enzyme having transgalactosylating activity for 3h at 65°C or 24h at 50, 55 or 60°C, wherein the enzyme is a Bifidobacterium bifidum β-galactosidase having the sequence of SEQ. ID. NO: 1 which was formulated with 60 w/w glucose and incubated for 69.5h at 55°C;
(c) fully or partly inactivating the enzyme, and
(d) thereby obtaining a milk product wherein at least 35wt% of the total free carbohydrates is in situ produced DP3+ galactooligosaccharides (GOS).

**2.** The milk product of claim 1, wherein at least 50 wt%, preferably at least 55 wt% or at least 60 wt%, of the total free carbohydrates is *in situ* produced DP2+ GOS.

**Patentansprüche**

**1.** Milchprodukt,

das eine Gesamtmenge von mindestens 11 Gew.-% an *in situ* produzierten DP3+ Galactooligosacchariden (GOS) umfasst und
wobei die *in situ* produzierten DP3+ GOS mindestens 35 Gew.-% der gesamten freien Kohlenhydrate ausmachen,
das Milchprodukt in einem Verfahren produziert wird, das Folgendes umfasst:

(a) Bereitstellen eines Milchsubstrats, das 60 % Magermilchpulverlösung umfasst, die aus Magermilchpulver hergestellt wird, das 50 % Laktose und 35 % Protein enthält;
(b) Inkontaktbringen des Milchsubstrats mit einem Enzym mit transgalactosylierender Aktivität für 3 Stunden bei 65 °C oder 24 Stunden bei 50, 55 oder 60 °C, wobei das Enzym eine Bifidobacterium bifidum β-13-Galactosidase mit der Sequenz von SEQ. ID. NR.: 1 ist, das mit 60 Gew./Gew. Glukose formuliert und für 69,5 Stunden bei 55 °C inkubiert wurde;
(c) vollständiges oder teilweises Inaktivieren des Enzyms und
(d) Erhalten dadurch von einem Milchprodukt, bei dem mindestens 35 Gew.-% der gesamten freien Kohlenhydrate *in situ* produzierte DP3+ -Galactooligosaccharide (GOS) sind.

**2.** Milchprodukt nach Anspruch 1, wobei mindestens 50 Gew.-%, vorzugsweise mindestens 55 Gew.-% oder mindestens 60 Gew.-% der gesamten freien Kohlenhydrate *in situ* produzierte DP2+ GOS sind.

**Revendications**

**1.** Produit laitier

qui comprend une quantité totale d'au moins 11 % en poids de galactooligosaccharides (GOS) DP3+ produits *in situ* et
dans lequel les GOS DP3+ produits *in situ* représentent au moins 35 % en poids des hydrates de carbone libres totaux,
ledit produit laitier est produit selon un procédé comprenant :

(a) la fourniture d'un substrat laitier comprenant une solution de lait écrémé en poudre à 60 % préparée à partir de lait écrémé en poudre contenant 50 % de lactose et 35 % de protéines ;
(b) la mise en contact du substrat laitier avec une enzyme ayant une activité de transgalactosylation pendant 3h à 65°C ou 24h à 50, 55 ou 60°C, l'enzyme étant une 13-galactosidase de Bifidobacterium bifidum ayant la séquence de SEQ. ID. N° 1 qui a été formulé avec 60 p/p de glucose et incubé pendant 69,5h à 55°C ;
(c) l'inactivation totale ou partielle de l'enzyme, et
(d) ainsi l'obtention d'un produit laitier dans lequel au moins 35 % en poids des glucides libres totaux sont des galactooligosaccharides (GOS) DP3+ produits *in situ*.

**2.** Produit laitier selon la revendication 1, dans lequel au moins 50 % en poids, de préférence au moins 55 % en poids ou au moins 60 % en poids, des hydrates de carbone libres totaux sont des GOS DP2+ produits *in situ*.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0458358 A1
- WO 2015086746 A

### Non-patent literature cited in the description

- **CHEN et al.** Optimization of the enzymic process for manufacturing low-lactose milk containing oligosaccharides. *Process Biochemistry*, 2002, vol. 38, 801-808
- **NEEDLEMAN ; WUNSCH**. *J. Mol. Biol.*, 1970, vol. 48, 443-453
- **RICE et al.** EMBOSS: The European Molecular Biology Open Software Suite. *Trends Genet*, 2000, vol. 16, 276-277
- *CHEMICAL ABSTRACTS*, 1310-73-2
- *CHEMICAL ABSTRACTS*, 10039-26-6